# FASCICULE DE BREVET EUROPEEN

(11) **EP 2 501 702 B1**
(45) Date de publication et mention de la délivrance du brevet: **06.08.2014**
(21) Numéro de dépôt: 10799083.0
(22) Date de dépôt: 19.11.2010
(51) Int. Cl.: C07D 495/04

(54) **PROCEDE DE PREPARATION DU N-BIOTINYL-6-AMINOCAPROATE DE N SUCCINIMIDYLE**
VERFAHREN ZUR HERSTELLUNG VON N-SUCCINIMIDYL-N-BIOTINYL-6-AMINOCAPROAT
METHOD FOR PREPARING N-SUCCINIMIDYL N-BIOTINYL-6-AMINOCAPROATE

(30) Priorité: 20.11.2009 FR 0905573
(43) Date de publication de la demande: 26.09.2012
(73) Titulaire: SANOFI, 75008 Paris (FR)
(72) Inventeur: POTIER, Pierre, F-75013 Paris (FR); SOLE, Raphaël, F-75013 Paris (FR); TROUILLEUX, Patrick, F-75013 Paris (FR)
(74) Mandataire: Jacobson, Claude
(86) Numéro de dépôt international: PCT/FR2010/052452
(87) Numéro de publication internationale: WO 2011/061449

(56) Documents cités:
- WO-A1-2006/021553
- ISLAM, I. ET AL.: "Evaluation of a Vitamin-Cloaking Strategy for Oligopeptide Therapeutics: Bionylated HIV-1 Protease Inhibitors", JOURNAL OF MEDICINAL CHEMISTRY, vol. 37, 1994, pages 293-304, XP002575371,

## Description

La présente invention se rapporte à un nouveau procédé de préparation du réactif de biotinylation N-biotinyl-6-aminocaproate de N-succinimidyle, ainsi qu'à un procédé de préparation de polysaccharides biotinylés mettant en oeuvre un tel réactif.

Le N-biotinyl-6-aminocaproate de N-succinimidyle est un réactif de biotinylation disponible commercialement, utilisé notamment pour le greffage du groupe biotine sur des molécules d'ADN ou des glycoprotéines.

Compte tenu des coûts de main d'oeuvre et des matières premières, et pour une obtention du N-biotinyl-6-aminocaproate de N-succinimidyle à l'échelle industrielle, il est nécessaire d'envisager une synthèse robuste, adaptée à la production du composé souhaité en grandes quantités et permettant d'obtenir ce dernier sous forme suffisamment pure, c'est-à-dire sans formation de sous-produits indésirables. Les Inventeurs ont maintenant trouvé une voie d'accès au N-biotinyl-6-aminocaproate de N-succinimidyle en deux étapes principales à partir de la biotine, qui satisfait aux besoins sus-mentionnés.

Le procédé selon l'invention comprend les étapes représentées ci-dessous dans le schéma 1, dans lequel R représente un groupe alkyle ou aryle et X et X', identiques ou différents l'un de l'autre, représentent des atomes d'halogène.

Dans le schéma 1, les composés de départ, intermédiaires et final sont les suivants :
- composé (I) : N-biotinyl-6-aminocaproate de N-succinimidyle,
- composé (II) : acide N-biotinyl-6-aminocaproïque,
- composé (II') : carboxylate de N-biotinyl-6-aminocaproate,
- composé (III) : biotine,
- composé (III') : biotinate de cyanométhyle.

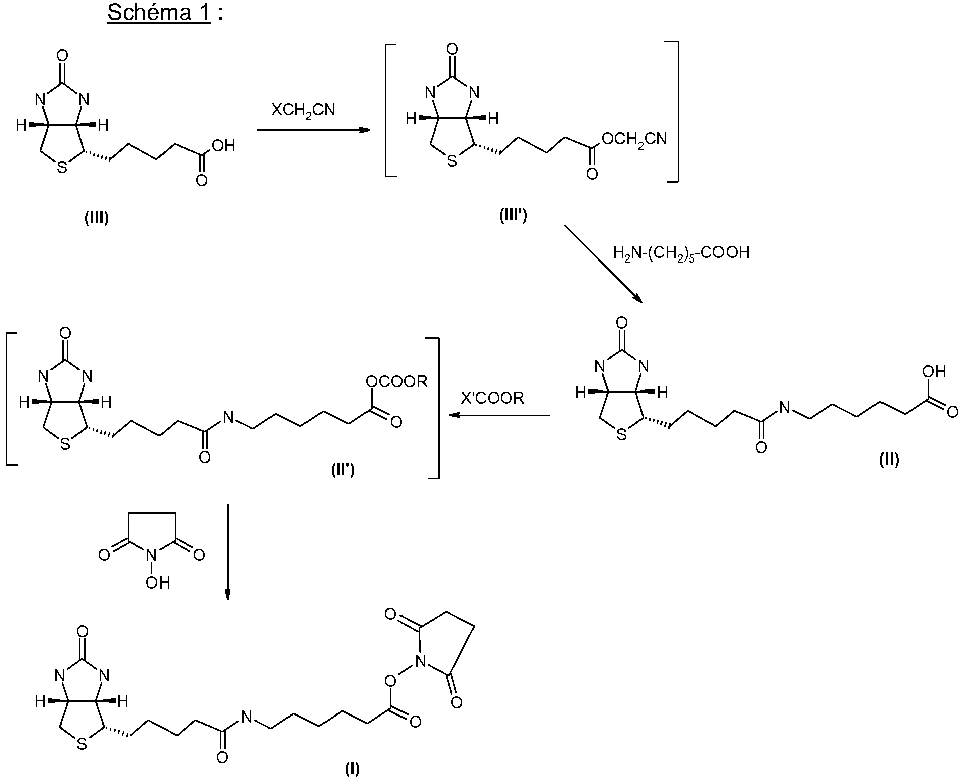

Selon la présente invention, et sauf mention différente dans le texte, on entend par:
- « alkyle » : un groupe aliphatique hydrocarboné saturé ou insaturé, linéaire ou ramifié, comprenant par exemple de 1 à 6 atomes de carbone, avantageusement de 1 à 4 atomes de carbone,
- « aryle » : un groupe aromatique cyclique, par exemple un groupe phényle, et
- « halogène » : un atome de fluor, chlore, brome ou iode.

L'invention a ainsi pour objet un procédé de préparation du composé (I), caractérisé en ce qu'il comprend une étape de couplage du composé (II') avec le N-hydroxysuccinimide. Ce couplage est avantageusement réalisé à une température inférieure à 0°C, par exemple à environ -5°C.

Selon l'invention, le composé (II') est obtenu en traitant le composé (II) par un halogénoformiate d'alkyle ou d'aryle (X'COOR, où R représente un groupe alkyle ou aryle et X un atome d'halogène) en présence d'une base faible de type amine tertiaire, dans un solvant polaire et aprotique, par exemple le DMF (diméthylformamide), le DMSO (diméthylsulfoxyde) ou la NMP (N-méthylpyrrolidone). Cette réaction permet d'activer, sous forme d'anhydride mixte, la fonction acide du composé (II). Elle est avantageusement réalisée à une température inférieure à 0°C, par exemple à environ -5°C.

Pour l'obtention du composé (II'), on utilise avantageusement le chloroformiate d'éthyle (CICOOEt). La réaction est avantageusement réalisée en présence de triéthylamine (NEt₃) en tant que base faible de type amine tertiaire, et dans un solvant tel que le DMF.

Selon l'invention, le composé (II) est obtenu par une étape de couplage entre le composé (III') et l'acide aminocaproïque (H₂N-(CH₂)₅-COOH). Lors de cette étape, on utilise avantageusement un solvant polaire, par exemple le DMF, le DMSO ou la NMP. La réaction est avantageusement effectuée à chaud, à environ 100°C.

Quant au composé (III'), il est obtenu en traitant le composé (III) par un halogénoacétonitrile (X-CH₂CN, où X représente un atome d'halogène), en présence d'une base faible de type amine tertiaire, dans un solvant polaire et aprotique, par exemple le DMF, le DMSO ou la NMP. Cette réaction permet d'activer, sous forme d'ester, la fonction acide du composé (III). La réaction est avantageusement effectuée à chaud, à environ 60°C.

Pour l'obtention du composé (III'), on utilise de préférence le chloroacétonitrile (CICH₂CN). La réaction est avantageusement réalisée en présence de triéthylamine en tant que base faible de type amine tertiaire, et dans un solvant tel que la NMP.

Selon le procédé de la présente invention, les intermédiaires de synthèse (II') et (III') ne sont pas isolés ; aussi la réaction de préparation du composé (I) présentée selon le schéma 1 consiste en une réaction en deux étapes à partir de la biotine.

De façon particulièrement avantageuse, le procédé selon l'invention permet d'accéder facilement au composé (II) en une étape à partir de la biotine et de l'acide aminocaproïque, avec un rendement élevé (supérieur à 80%) et aboutissant à un produit d'une excellente pureté.

De façon surprenante, d'autres voies d'accès au composé (II) qui auraient pu être envisagées, inspirées des techniques de couplage issues de la synthèse peptidique, se sont révélées inefficaces ou inadaptées à la production industrielle.

En particulier, les Inventeurs ont montré que la voie au chlorure d'acide, qui consiste à transformer la biotine (III) en chlorure d'acide (par exemple D.E. Wolf et al. dans J. Am. Chem. Soc., année 1951, 73, p. 4142-4144 et année 1952, 74, p. 2002-2003), puis à la faire réagir avec l'acide aminocaproïque en milieu basique, ne permet pas un rendement satisfaisant, du fait de l'insolubilité de la biotine. Par ailleurs, cette voie implique l'utilisation de chlorure de thionyle en très large excès : la toxicité de ce solvant cause des problèmes d'hygiène industrielle.

Quant à la voie au dicyclohexylcarbodiimide (DCC), qui consiste à activer *in situ* la biotine avant son couplage avec l'acide aminocaproïque (voir par exemple C. Somlai et al. dans Zeit. Naturforsch., 1993, 48, p. 511-516), elle s'est révélée inefficace de par la réactivité insuffisante entre la biotine activée et l'acide aminocaproïque. En outre, l'utilisation du DCC avec l'acide aminocaproïque génère du DCU (dicyclohexylurée), sous-produit très difficilement éliminable en fin de réaction (DCU inséparable du composé (II')).

Enfin, la voie aux anhydrides mixtes (activation de la fonction acide de la biotine par la formation d'un anhydride très réactif, qui réagit ensuite avec l'acide aminocaproïque) permet d'obtenir le composé désiré (II), mais présente plusieurs inconvénients qui la rende inadaptée pour l'échelle industrielle : en effet, quelles que soient les conditions utilisées, il reste toujours de la biotine en fin de réaction (au moins 2%), il se forme un produit secondaire correspondant à l'addition de 2 acides aminocaproïques sur la biotine, et le milieu se transforme rapidement en gel extrêmement épais.

La voie aux esters activés selon la présente invention surmonte donc les inconvénients des autres voies précitées pour l'obtention du composé (II).

De façon particulièrement avantageuse, le procédé selon l'invention permet d'accéder au composé (I) en une étape à partir de l'intermédiaire de formule (II) et du N-hydroxysuccinimide, selon la voie de synthèse aux anhydrides mixtes. Cette voie de synthèse permet d'obtenir le composé (I) avec une pureté et un rendement élevés.

L'invention a également pour objet un procédé de préparation de polysaccharides biotinylés, par exemple d'idrabiotaparinux, caractérisé en ce qu'il comprend les étapes suivantes :
- préparation du composé (I) selon le procédé défini ci-avant, puis
- couplage du composé (I) avec un polysaccharide présentant une fonction réactive envers la fonction ester activé du composé (I).

Ladite fonction réactive du polysaccharide est par exemple une fonction amine, auquel cas le couplage avec le composé (I), qui comprend une fonction ester activé, est effectué par une réaction classique de couplage amino/acide.

La réaction de couplage entre le composé (I) et le polysaccharide est avantageusement réalisée dans une solution aqueuse d'hydrogénocarbonate de sodium.

Les polysaccharides biotinylés dont la préparation est décrite ci-dessus sont par exemple tels que ceux décrits dans les demandes de brevets WO 02/24754 et WO 2006/030104. Il peut s'agir notamment du pentasaccharide biotinylé connu sous la Dénomination Commune Internationale « idrabiotaparinux » et décrit dans la demande de brevet WO 02/24754, ou des hexadécasaccharides biotinylés décrits dans les exemples 1 et 2 de la demande de brevet WO 2006/030104.

Pour préparer ces polysaccharides biotinylés, le composé (I) est couplé, respectivement, avec le pentasaccharide 44 décrit dans la demande de brevet WO 02/24754 ou avec les hexadécasaccharides 42 et 43 décrits dans la demande de brevet WO 2006/030104 :
- pentasaccharide 44 : méthyl (2-amino-2-désoxy-3,4-di-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyl-uronique)-(1→4)-2,3,6-tri-O-sulfonato-α-D-glucopyranoside ;
- hexadécasaccharide 42 : méthyl (2,3,4,6-tétra-O-sulfonato-a-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-amino-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-3-O-méthyl-2,6-di-O-sulfonato-α-D-glucopyranoside ;
- hexadécasaccharides 43 : méthyl (2,3,4,6-tétra-O-sulfonato-α-D-glucopyranosyl)-(1→4)-(2,3,6-tri-O-sulfonato-α-D-glucopyranosyl)-(1→4)-(2,3,6-tri-O-sulfonato-β-D-glucopyranosyl)-(1→4)-(2,3-di-O-méthyl-6-O-sulfonato-α-D-glucopyranosyl)-(1→4)-(2,3,6-tri-O-méthyl-β-D-glucopyranosyl)-(1→4)-[(2,3,6-tri-O-méthyl-α-D-glucopyranosyl)-(1→4)-O-(2,3,6-tri-O-méthyl-β-D-glucopyranosyl)-(1→4)]₃-(2-amino-2-désoxy-3-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyluronique)-(1→4)-2,3,6-tri-O-sulfonato-α-D-glucopyranoside.

L'invention est illustrée à l'aide des exemples qui suivent, qui détaillent un procédé de préparation du composé (I) conformément à l'invention. Dans ces exemples, on utilise les abréviations suivantes : Ac : acétyle ; CPL : Chromatographie en Phase Liquide ; DMF : diméthylformamide ; min : minutes ; MTBE : méthyl-tert-butyl-éther ; NMP : N-méthylpyrrolidone ; éq : équivalents molaires ; Et : éthyle ; T : température ; THF : tétrahydrofurane ; tr : temps de rétention ; UO : unités opératoires, V : volume.

### Exemple 1 : Préparation du composé (I)

Le suivi des réactions se fait par CPL avec les conditions suivantes : colonne Symmetry C18 150 x 4.6 mm 5µ (Waters) ; éluant A : tampon KH₂PO₄ 0.01 M ajusté pH = 3 ; éluant B : acétonitrile ; débit 1 mL/min ; gradient : t = 0 min A/B 85/15, t = 9 min A/B 65/35, t = 10 min A/B 85/15, t = 15 min A/B 85/15. Cette méthode permet de visualiser la biotine (composé (III), tr = 4.5 min), l'ester activé intermédiaire (III') (tr = 8.4 min), l'acide N-biotinyl-6-aminocaproïque (composé (II), tr = 5.5 à 5.6 min), l'anhydride mixte intermédiaire (II') (tr = 11.2 min) et le N-biotinyl-6-aminocaproate de N-succinimidyle (composé (I), tr = 7.9 à 8.2 min).

### 1.1 : Préparation du composé (II)

Dans un réacteur, on charge 7.5 kg de biotine (III), de la triéthylamine (15 L, 2 V, 3.5 éq), de la NMP (15 L, 2 V), et enfin le chloroacétonitrile (3.5 kg, 0.47 UO, 1.5 éq). Le milieu est chauffé à 60°C. Après 2h de maintien à cette température, une analyse CPL montre que toute la biotine a été transformée en composé (III') (<2%). Le milieu est refroidi à 50°C puis transféré dans un autre réacteur contenant l'acide aminocaproïque (9.05 kg, 1.206 UO, 2.2 éq). On rincer avec de la NMP (0.1 V). Le milieu est chauffé à 100°C et maintenu à cette température pendant 2h. Une analyse CPL montre qu'il reste moins de 2% de biotine activée (III'). Le milieu est refroidi à 60°C. On coule de l'acétonitrile (60 L, 8 V) préalablement chauffé à 55°C. On agite 30 min à 60°C, puis on refroidit à 20°C. On agite pendant 1 h. On filtre la suspension, puis on rince par 3 fois de l'acétonitrile (5 V), puis par du THF (5 V). On sèche sous vide à 60°C maximum jusqu'à poids constant. On obtient ainsi 12.0 kg du composé (II), avec un rendement de 109% et une pureté organique, mesurée par CPL, de 98.6%.

10.0 kg du composé (II) est rechargé dans un réacteur. De l'acide chlorhydrique (eau 90 L, 9 V + HCl 36% 10 L, 1 V) est alors ajouté. On agite la suspension à 20°C pendant 30 min. On filtre et on rince 3 fois par de l'eau (4 V, 40 L), puis 2 fois par du THF (3.5 V). On sèche sous vide à 45°C maximum jusqu'à poids constant. On obtient ainsi 6.1 kg du composé (II), avec un rendement de 66%.

### 1.2 : Préparation du composé (I)

Dans un réacteur, on met en suspension 3 kg du composé (II) dans le DMF (25 L, 8.3 V) et on amène la température à -5°C. La triéthylamine (1.02 kg, 0.34 UO, 1.2 éq) est alors ajoutée. Après 15 minutes d'agitation, le chloroformiate d'éthyle (1.1 kg, 0.365 UO, 1.2 éq) est ajouté doucement (en au moins 1 h). On rince par du DMF (0.9 L, 0.3 V). Le milieu est agité à -5°C pendant au moins 2h. La suspension devient plus fine et jaune. Une analyse CPL montre que tout le composé (II) (<3%) a réagi.

Le N-hydroxysuccinimide (1.04 kg, 0.386 UO, 1.2 éq) en solution dans le DMF (3 L, 1 V) est alors introduit en 1 fois (en au moins 20 min). On rince par du DMF (1.5 L, 0.5 V). Le milieu est agité pendant 1h30 à -5°C. Une analyse CPL montre que la présence de composé (II) résiduelle est inférieure à 3%. La température est amenée à 22°C, la suspension est reprise dans du DCM (12 V, 36 L) et la phase organique résultante est lavée avec de l'eau (15 L, 5 V). La phase organique est soutirée et la phase aqueuse est extraite 2 fois avec du DCM (30 L, 3 V). On mélange les phases organiques et on les lave avec de l'eau (1.5 L, 0.5 V). On concentre la phase organique à 6 V, soit 18 L. On chauffe à 40°C et on additionne le MTBE (6.25 V, 19 L) en 1 h minimum. On maintient 1 h à 40°C, puis on additionne du MTBE (8.75 V, 26 L) en 2h minimum. On maintient au moins 30 minutes à 40°C, puis on refroidit à 20°C en 2h minimum, et on maintient 30 minutes à cette température. On essore la suspension et on lave le gâteau avec de l'acétone (5 V, 15 L), puis encore 2 fois avec de l'acétone (2 V, 6 L). On essore et on sèche à l'étuve sous vide à 40°C maximum jusqu'à poids constant.

3 kg du composé (I) sont ainsi obtenus sous la forme d'une poudre crème, avec un rendement de 80% et une pureté organique, mesurée par CPL, de 96.0%. Hors composé (II), dont la présence n'est pas gênante pour une réaction ultérieure de couplage avec un polysaccharide puisqu'il sera inerte lors de ce couplage, la pureté du composé (I) est de 98%.

### Exemple 2 : Préparation d'un polysaccharide biotinylé, l'idrabiotaparinux

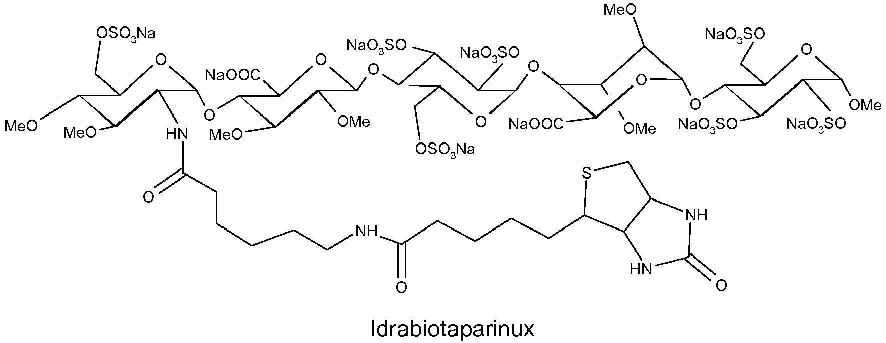

On prépare une solution de 1.22 kg du pentasaccharide 44 brut (contenant des sels), tel que décrit dans la demande de brevet WO 02/24754, dans 8.5 L d'eau (7 V). On y ajoute 0.5 kg (1.6 éq) du composé (I), 0.12 kg (2.0 éq) de NaHCO₃ et 0.37 kg de NaCl. La solution se présente sous la forme d'une suspension blanche. On y ajoute 3.7 L d'acétone et on agite le milieu réactionnel à environ 25°C pendant au moins 22h. On coule ensuite lentement cette suspension sur un mélange d'éthanol (120 L) et MTBE (60 L) préalablement refroidi à environ 4°C, ce qui permet de faire précipiter le pentasaccharide biotinylé. On filtre ensuite la suspension ainsi obtenue et on la rince successivement avec de l'éthanol absolu et de l'acétone. On sèche le précipité à l'étuve sous vide jusqu'à poids constant. On obtient ainsi 1.60 kg d'idrabiotaparinux brut (contenant des sels) sous la forme d'une poudre crème, avec une pureté organique de 99%, avec un rendement de 109% par rapport au pentasaccharide 44 et un rendement chimique de 70% sur les 3 dernières étapes.

## Revendications

1. Procédé de préparation du N-biotinyl-6-aminocaproate de N-succinimidyle, répondant à la formule (I) et comprenant une fonction ester activé : 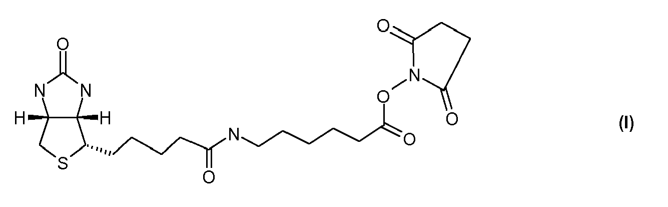 **caractérisé en ce qu'**il comprend une étape d'activation, sous forme d'anhydride mixte, de la fonction acide du composé (II) : 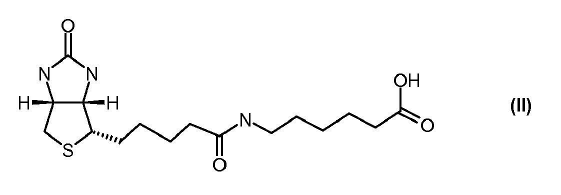 de façon à obtenir le composé (II'), dans lequel R représente un groupe alkyle ou aryle : 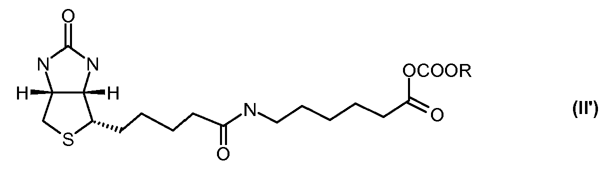 suivie du couplage du composé (II') avec le N-hydroxysuccinimide.

2. Procédé selon la revendication 1, **caractérisé en ce que** l'activation de la fonction acide du composé (II) est réalisée en traitant ledit composé par un halogénoformiate d'alkyle ou d'aryle de formule X'COOR, où R représente un groupe alkyle ou aryle et X' représente un atome d'halogène, en présence d'une base faible de type amine tertiaire, dans un solvant polaire et aprotique.

3. Procédé selon la revendication 2, **caractérisé en ce que** ledit halogénoformiate de formule X'COOR est le chloroformiate d'éthyle.

4. Procédé selon la revendication 2 ou 3, **caractérisé en ce que** ladite base faible de type amine tertiaire est la triéthylamine.

5. Procédé selon l'une quelconque des revendications 2 à 4, **caractérisé en ce que** ledit solvant polaire et aprotique est choisi parmi le diméthylformamide, le diméthylsulfoxyde ou la N-méthylpyrrolidone.

6. Procédé selon l'une quelconque des revendications 1 à 5, **caractérisé en ce que** l'étape d'activation de la fonction acide du composé (II) est réalisée à une température inférieure à 0°C.

7. Procédé selon l'une quelconque des revendications 1 à 6, **caractérisé en ce que** le composé (II) est obtenu par une étape d'activation, sous forme d'ester, la fonction acide du composé (III) : 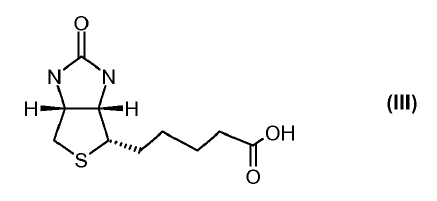 de façon à obtenir le composé (III') : 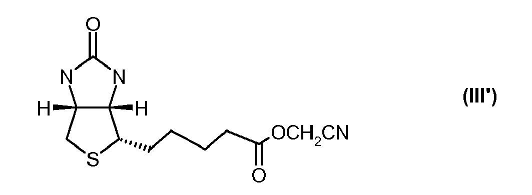 suivie du couplage du composé (III') avec l'acide aminocaproïque.

8. Procédé selon la revendication 7, **caractérisé en ce que** l'activation de la fonction acide du composé (III) est réalisée à l'aide d'un halogénoacétonitrile, en présence d'une base faible de type amine tertiaire, dans un solvant polaire et aprotique.

9. Procédé selon la revendication 8, **caractérisé en ce que** ledit halogénoacétonitrile est le chloroacétonitrile.

10. Procédé selon la revendication 8 ou la revendication 9, **caractérisé en ce que** ladite base faible de type amine tertiaire est la triéthylamine.

11. Procédé selon l'une quelconque des revendications 8 à 10, **caractérisé en ce que** ledit solvant polaire et aprotique est choisi parmi le diméthylformamide, le diméthylsulfoxyde ou la N-méthylpyrrolidone.

12. Procédé selon l'une quelconque des revendications 7 à 11, **caractérisé en ce que** la réaction d'activation de la fonction acide du composé (III) est réalisée à environ 60°C.

13. Procédé selon l'une quelconque des revendications 7 à 12, **caractérisé en ce que** la réaction de couplage entre le composé (III') et l'acide aminocaproïque est réalisée à environ 100°C.

14. Procédé de préparation de polysaccharides biotinylés, **caractérisé en ce qu'**il comprend les étapes suivantes :
- préparation du composé (I) selon le procédé défini dans l'une quelconque des revendications 1 à 13, puis
- couplage du composé (I) avec un polysaccharide présentant une fonction réactive envers la fonction ester activé du composé (I).

15. Procédé selon la revendication 14, **caractérisé en ce que** ladite fonction réactive du polysaccharide est une fonction amine.

16. Procédé selon la revendication 14 ou la revendication 15, **caractérisé en ce qu'**il consiste en la préparation d'idrabiotaparinux et qu'il comprend les étapes suivantes :
- préparation du composé (I) selon le procédé défini dans l'une quelconque des revendications 1 à 13, puis
- couplage du composé (I) avec le sel de sodium du pentasaccharide méthyl (2-amino-2-désoxy-3,4-di-O-méthyl-6-O-sulfonato-α-D-glucopyranosyl)-(1→4)-(acide 2,3-di-O-méthyl-β-D-glucopyranosyluronique)-(1→4)-(2,3,6-tri-O-sulfonato-α-D-glucopyranosyl)-(1→4)-(acide 2,3-di-O-méthyl-α-L-idopyranosyl-uronique)-(1→4)-2,3,6-tri-O-sulfonato-α-D-glucopyranoside.

## Patentansprüche

1. Verfahren zur Herstellung von N-Biotinyl-6-amino-capronsäure-N-succinimidylester, der der Formel (I) entspricht und eine aktivierte Esterfunktion umfasst: 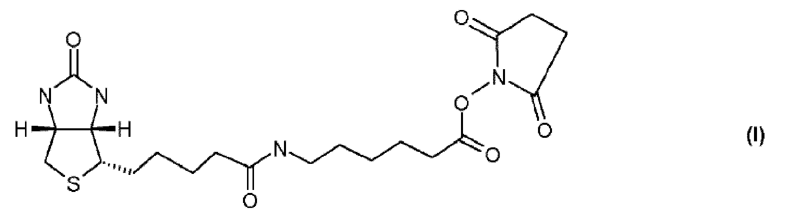 **dadurch gekennzeichnet, dass** es einen Schritt der Aktivierung, in Form eines gemischten Anhydrids, der der Säurefunktion der Verbindung (II) : 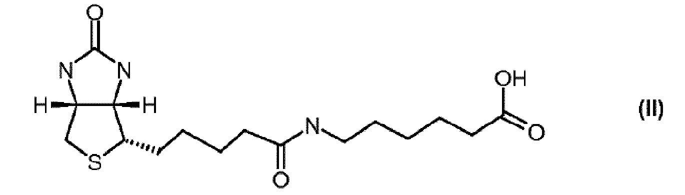 unter Erhalt der Verbindung (II'), in der R für eine Alkyl- oder Arylgruppe steht: 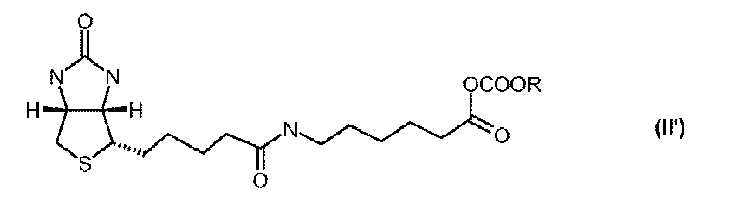 gefolgt von der Kupplung der Verbindung (II') mit N-Hydroxysuccinimid umfasst.

2. Verfahren nach Anspruch 1, **dadurch gekennzeichnet, dass** die Aktivierung der Säurefunktion der Verbindung (II) durch Behandlung der Verbindung mit einem Halogenameisensäurealkylester oder -arylester der Formel X'COOR, wobei R für eine Alkyl- oder Arylgruppe steht und X' für ein Halogenatom steht, in Gegenwart einer schwachen Base vom Typ tertiäres Amin in einem polaren und aprotischen Lösungsmittel erfolgt.

3. Verfahren nach Anspruch 2, **dadurch gekennzeichnet, dass** es sich bei dem Halogenameisensäureester der Formel X'COOR um Chlorameisensäureethylester handelt.

4. Verfahren nach Anspruch 2 oder 3, **dadurch gekennzeichnet, dass** es sich bei der schwachen Base vom Typ tertiäres Amin um Triethylamin handelt.

5. Verfahren nach einem der Ansprüche 2 bis 4, **dadurch gekennzeichnet, dass** man das polare und aprotische Lösungsmittel aus Dimethylformamid, Dimethylsulfoxid oder N-Methylpyrrolidon auswählt.

6. Verfahren nach einem der Ansprüche 1 bis 5, **dadurch gekennzeichnet, dass** der Schritt der Aktivierung der Säurefunktion der Verbindung (II) bei einer Temperatur von weniger als 0°C erfolgt.

7. Verfahren nach einem der Ansprüche 1 bis 6, **dadurch gekennzeichnet, dass** man die Verbindung (II) durch einen Schritt der Aktivierung, in Form eines Esters, der der Säurefunktion der Verbindung (III): 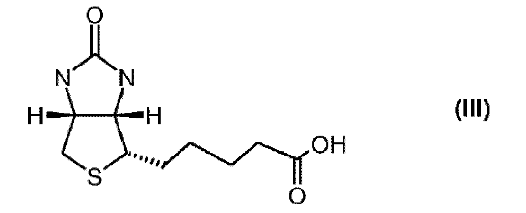 unter Erhalt der Verbindung (III') : 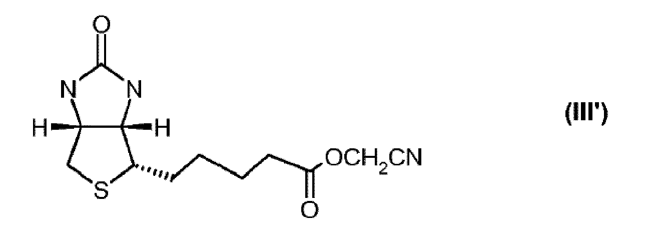 gefolgt von der Kupplung der Verbindung (III') mit Aminocapronsäure erhält.

8. Verfahren nach Anspruch 7, **dadurch gekennzeichnet, dass** die Aktivierung der Säurefunktion der Verbindung (III) mit Hilfe eines Halogenacetonitrils in Gegenwart einer schwachen Base vom Typ tertiäres Amin in einem polaren und aprotischen Lösungsmittel erfolgt.

9. Verfahren nach Anspruch 8, **dadurch gekennzeichnet, dass** es sich bei dem Halogenacetonitril um Chloracetonitril handelt.

10. Verfahren nach Anspruch 8 oder Anspruch 9, **dadurch gekennzeichnet, dass** es sich bei der schwachen Base vom Typ tertiäres Amin um Triethylamin handelt.

11. Verfahren nach einem der Ansprüche 8 bis 10, **dadurch gekennzeichnet, dass** man das polare und aprotische Lösungsmittel aus Dimethylformamid, Dimethylsulfoxid oder N-Methylpyrrolidon auswählt.

12. Verfahren nach einem der Ansprüche 7 bis 11, **dadurch gekennzeichnet, dass** die Aktivierung der Säurefunktion der Verbindung (III) bei ungefähr 60°C erfolgt.

13. Verfahren nach einem der Ansprüche 7 bis 12, **dadurch gekennzeichnet, dass** die Kupplungsreaktion zwischen der Verbindung (III') und Aminocapronsäure bei ungefähr 100°C erfolgt.

14. Verfahren zur Herstellung von biotinylierten Polysacchariden, **dadurch gekennzeichnet, dass** es die folgenden Schritte umfasst:
- Herstellung der Verbindung (I) gemäß dem in einem der Ansprüche 1 bis 13 definierten Verfahren, dann
- Kupplung der Verbindung (I) mit einem Polysaccharid mit einer Funktion, die gegenüber der aktivierten Esterfunktion der Verbindung (I) reaktiv ist.

15. Verfahren nach Anspruch 14, **dadurch gekennzeichnet, dass** es sich bei der reaktiven Funktion des Polysaccharids um eine Aminfunktion handelt.

16. Verfahren nach Anspruch 14 oder Anspruch 15, **dadurch gekennzeichnet, dass** es aus der Herstellung von Idrabiotaparinux besteht und die folgenden Schritte umfasst:
- Herstellung der Verbindung (I) gemäß dem in einem der Ansprüche 1 bis 13 definierten Verfahren, dann
- Kupplung der Verbindung (I) mit dem Natriumsalz des Pentasaccharids Methyl-(2-amino-2-desoxy-3,4di-O-methyl-6-O-sulfonato-α-D-glucopyranosyl)-(1→4)-(2,3-di-O-methyl-β-D-glucopyranosyluronsäure)-(1→4)-(2,3,6-tri-O-sulfonato-α-D-glucopyranosyl)-(1→4)-(2,3-di-O-methyl-α-L-idopyranosyluronsäure)-(1→4)-2,3,6-tri-O-sulfonato-α-D-glucopyranosid.

## Claims

1. A process for preparing N-succinimidyl N-biotinyl-6-aminocaproate corresponding to formula (I) and comprising an activated ester function: 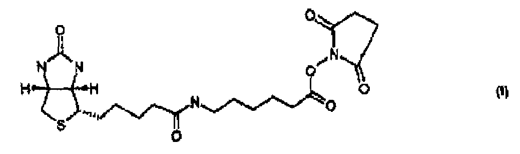 **characterized in that** it comprises a stage of activating, in the form of a mixed anhydride, the acid function of the compound (II): 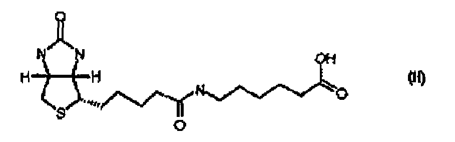 so as to obtain the compound (II'), in which R represents an alkyl or aryl group: 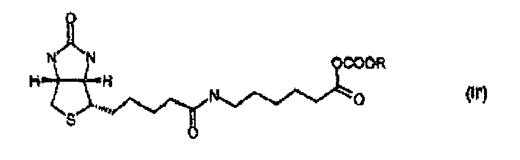 followed by coupling of the compound (II') with N-hydroxysuccinimide.

2. The process as claimed in claim 1, **characterized in that** the activation of the acid function of the compound (II) is carried out by treating said compound with an alkyl or aryl haloformate of formula X'COOR, where R represents an alkyl or aryl group and X' represents a halogen atom, in the presence of a weak base of tertiary amine type, in a polar and aprotic solvent

3. The process as claimed in claim 2, **characterized in that** said haloformate of formula X'COOR is ethyl chloroformate.

4. The process as claimed in claim 2 or 3, **characterized in that** said weak base of tertiary amine type is triethylamine.

5. The process as claimed in any one of claims 2 to 4, **characterized in that** said polar and aprotic solvent is selected from dimethylformamide, dimethyl sulfoxide or N-methylpyrrolidone,

6. The process as claimed in any one of claims 1 to 5, **characterized in that** the stage of activating the acid function of the compound (II) is carried out at a temperature below 0°C.

7. The process as claimed in any one of claims 1 to 6, **characterized in that** the compound (II) is obtained by means of a step of activating, in the form of an ester, the acid function of the compound (III): 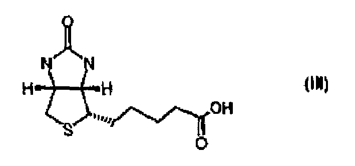 so as to obtain the compound (III'): 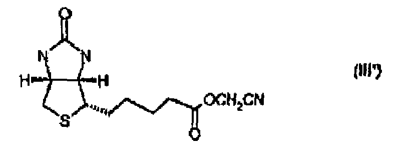 followed by coupling of the compound (III') with aminocaproic acid.

8. The process as claimed in claim 7, **characterized in that** the activation of the acid function of the compound (III) is carried out using a haloacetonitrile, in the presence of a weak base of tertiary amine type, in a polar and aprotic solvent.

9. The process as claimed in claim 8, **characterized in that** said haloacetonitrile is chloroacetonitrile.

10. The process as claimed in claim 8 or claim 9, **characterized in that** said weak base of tertiary amine type is triethylamine.

11. The process as claimed in any one of claims 8 to 10, **characterized in that** said polar and aprotic solvent is selected from dimethylformamide, dimethyl sulfoxide or N-methylpyrrolidone.

12. The process as claimed in any one of claims 7 to 11, **characterized in that** the reaction for activation of the acid function of the compound (III) is carried out at approximately 60°C.

13. The process as claimed in any one of claims 7 to 12, **characterized in that** the coupling reaction between the compound (III') and aminocaproic acid is carried out at approximately 100°C.

14. A process for preparing biotinylated polysaccharides, **characterized in that** it comprises the following stages:
- preparing the compound (I) according to the process defined in any one of claims 1 to 13, then
- coupling the compound (I) with a polysaccharide which has a function that is reactive toward the activated ester function of the compound (I).

15. The process as claimed in claim 14, **characterized in that** said reactive function of the polysaccharide is an amine function.

16. The process as claimed in claim 14 or claim 15, **characterized in that** it consists of the preparation of idrabiotaparinux and **in that** it comprises the following stages:
- preparing the compound (I) according to the process defined in any one of claims 1 to 13, then
- coupling the compound (I) with the sodium salt of the pentasaccharide methyl (2-amino-2-deoxy-3,4-di-O-methyl-6-O-sulfonato-α-D-glucopyranosyl)-(1→4)-(2,3-di-O-methyl-β-D-glucapyranosyluronic acid)-(1→4)-(2,3,6-tri-O-sulfonato-α-D-glucopyranosyl)-(1→4)-(2,3-di-O-methyl-α-L-idopyranosyluronic acid)-(1→4)-2,3,6-tri-O-sulfonato-α-D-glucopyranoside.
